(19) **Europäisches Patentamt**

**European Patent Office**

**Office européen des brevets**

(11) **EP 0 979 811 A1**

(12) **EUROPÄISCHE PATENTANMELDUNG**

(43) Veröffentlichungstag:
16.02.2000  Patentblatt 2000/07

(51) Int. Cl.⁷: **C07C 45/53**, C07C 45/80,
C07C 45/79, C07C 37/08,
C07C 37/72, C07C 37/82

(21) Anmeldenummer: 99114766.1

(22) Anmeldetag: 28.07.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **05.08.1998 DE 19835306**
**08.01.1999 DE 19900382**

(71) Anmelder:
**Phenolchemie GmbH & Co. KG**
**45966 Gladbeck (DE)**

(72) Erfinder:
• **Pompetzki, Werner, Dr.**
**46284 Dorsten (DE)**
• **Gerlich, Otto, Dr.**
**45966 Galdbeck (DE)**

(54) **Verfahren zur Entfernung von organischen und/oder anorganischen Säuren aus organischen Phasen**

(57)  Es wird ein Verfahren zur Entfernung von organischen und/oder anorganischen Säuren aus organischen Phasen durch Anwendung einer Extraktion mit nachgeschaltetem Ionenaustauscher beansprucht. Der Vorteil der Kombination der beiden Verfahrensschritte liegt in der wesentlich geringeren Wassermenge die bei der Extraktion eingesetzt werden muß, da eine vollständige Entfernung der Säuren, wie dies bei einem einstufigen Verfahren notwendig ist, nicht nötig ist. Die in der organischen Phase verbleibende Säure wird mit einem handelsüblichen Ionenaustauscher entfernt.

Das erfindungsgemäße Verfahren wird unter anderem zur Entfernung von organischen und/oder anorganischen Säuren aus der bei der Phenolherstellung durch säurekatalysierte Spaltung von Cumolhydroperoxid anfallenden Spaltproduktphase genutzt.

**Beschreibung**

Die vorliegende Erfindung betritt ein Verfahren zur Entfernung von organischen und/oder anorganischen Säuren aus organischen Phasen.

Im besonderen betritt die vorliegende Erfindung ein Verfahren zur Entfernung von organischen und/oder anorganischen Säuren aus Phasen, die bei der Spaltung von Aralkylhydroperoxiden entstehen.

Bei vielen säurekatalysierten Prozessen verbleibt ein nicht unerheblicher Teil der als Katalysator eingesetzten organischen und/oder anorganischen Säuren in den entstehenden Gemischen. Die Gemische werden, wenn möglich, durch Phasentrennung in eine wäßrige und eine organische Phase getrennt, wobei in der organischen Phase immer noch anorganische Bestandteile wie z. B. Wasser und anorganische Säuren gelöst sind. Bei der Aufarbeitung dieser Produktphase kann die noch vorhandene Säure unerwünschte Nebenreaktionen wie z. B. säurekatalysierten Alkylierungs- oder Kondensationsreaktionen hervorrufen, die zu Ausbeuteverlusten an Wertprodukt führen. Um solche unerwünschten Nebenreaktionen zu unterbinden, ist es deshalb notwendig, daß die organischen und/oder anorganischen Säuren aus der organischen Phase möglichst schnell abgetrennt werden.

Ein Prozeß, der von großer wirtschaftlicher Bedeutung ist, ist der bei der Gewinnung von Phenol und Aceton ausgehend von Cumol nach dem klassischen Hock-Verfahren durchgeführte Prozeß der säurekatalysierten Spaltung von Cumolhydroperoxid zu Phenol und Aceton. Bei diesem Prozeß fällt bei der durch Schwefelsäure katalysierten Spaltreaktion von Cumolhydroperoxid (CHP) zu Phenol und Aceton eine saure, überwiegend organische Phase an, welche Schwefelsäure enthält. Hinzu kommen in der Regel organische Säuren, welche als Nebenprodukte bei der Oxidation von Cumol oder bei der säurekatalysierten Spaltung entstehen können, so daß der Gesamtsäuregehalt noch etwas höher liegt. Zur Vermeidung von säurekatalysierten Alkylierungs- und Kondensationsreaktionen im Spaltprodukt und zur Vermeidung von Korrosionsschäden an den eingesetzten Apparaten müssen vor der beispielsweise auf destillativem Wege erfolgenden Trennung des Spaltprodukts in seine Bestandteile die Säuren schnell aus dem Spaltprodukt entfernt werden.

Dies geschieht praktisch üblicherweise durch sofortige Neutralisation des bei der säurekatalysierten Spaltung von Cumolhydroperoxid entstehenden Spaltprodukts mit wäßrigen Lösungen von Alkalihydroxiden und/oder -phenolaten und anschließende Salzwäsche zur Entfernung des anfallenden Alkalisulfats (so beschrieben in EP 0 032 255, EP 0 085 289, BP 756 408, DE 1 128 859 und DE 25 12 842). Trotz Neutralisation und Salzwäsche bleibt jedoch noch eine nicht vernachlässigbare Menge an Alkalisulfat bzw. Alkalicarboxylaten im Spaltprodukt gelöst und/oder emulgiert. In der nachgeschalteten Destillation kommt es deswegen immer wieder zu Salzablagerungen, die ein regelmäßiges Spülen und Reinigen der Verdampfer erfordern. Der hohe Salzgehalt, der bei der Destillation im Sumpf der Destillationskolonne zurückbleibt, erschwert außerdem die Aufarbeitung des Rückstands, der bei der Destillation zurückbleibt, weshalb es notwendig ist, die Salze möglichst früh aus dem Spaltproduktgemisch abzutrennen.

Aus US 4 262 150 und US 4 262 151 ist weiterhin bekannt, daß durch gezielte Neutralisation des bei der säurekatalysierten Spaltung von Cumolhydroperoxid entstehenden Spaltproduktes unter Einhaltung eines pH-Wertes von 2 bis 6 und anschließende Salzwäsche eine Verbesserung der Salzabtrennung erzielt werden kann.

In US 5 510 543 wird eine weitere Möglichkeit zur Verbesserung der Salzabtrennung beschrieben. Diese wird erreicht durch Zugabe von 3 bis 5 Gew.-% Cumol zum Spaltproduktgemisch vor der Neutralisation.

Die Neutralisation des bei der säurekatalysierten Spaltung von Cumolhydroperoxid entstehenden Spaltproduktgemisches ist, wie in US 3 927 124 beschrieben, auch durch die Verwendung von Ammoniak, Methylamin oder Triethylamin möglich.

Die nach der Neutralisation notwendige Salzwäsche wird bei all diesen Verfahren oft dadurch erschwert, daß es bei der Salzwäsche des Gemisches zu nicht unerheblichen Phasentrennungprobleblemen kommen kann.

Aus der Patentschrift DE 963 520 ist daher auch die Entfernung der Schwefelsäure aus dem bei der säurekatalysierten Spaltung von Cumolhydroperoxid entstehenden sauren Spaltprodukt durch die Extraktion mit einer schwachen nicht flüchtigen Carbonsäure bekannt.

In DD 91 643 ist die Entfernung der Schwefelsäure aus dem bei der säurekatalysierten Spaltung von Cumolhydroperoxid entstehen sauren Spaltprodukt durch Extraktion mit Wasser beschrieben. Dabei sind jedoch erhebliche Wassermengen (15 bis 35 Gew.-%) erforderlich, um den Schwefelsäuregehalt auf unter 10 ppm im Spaltprodukt zu senken, die anschließend bearbeitet werden müssen, bevor sie dem Abwasser zugeführt werden können. Die Extraktion erfolgt in einer Extraktionskolonne mit 10 mm großen Raschigringen bei Temperaturen von 20 bis 40 °C. Dieses Verfahren ist auch auf ein teilneutralisiertes Spaltprodukt anwendbar.

Aus CA 697 600, FR 1 302 848 und SU 118 415 ist außerdem bekannt, daß die Entfernung von Schwefelsäure aus dem bei der säurekatalysierten Spaltung von Cumolhydroperoxid entstehenden Spaltprodukt ohne Extraktion durch den Einsatz von Anionenaustauschern möglich ist, wobei die Regeneration des Anionenaustauschers mit einer Alkalihydroxidlösung erfolgt. Der Einsatz der Ionenaustauscherharze zur Entfernung der relativ hoch konzentrierten Schwefelsäure bedingt jedoch, auf Grund der geringen Beständigkeit der Anionenaustauscherharze gegenüber dem Spaltprodukt, eine verhältnismäßig kurze Lebensdauer des Anionenaustauschers.

Der Erfindung liegt daher die Aufgabe zugrunde, ein einfaches und kostengünstiges Verfahren zur Entfernung von organischen und/oder anorganischen Säuren aus organischen Phasen bereitzustellen.

Im besonderen liegt der Erfindung die Aufgabe zugrunde, ein einfaches Verfahren zur Entfernung von organischen und/oder anorganischen Säuren aus Phasen, die bei der Spaltung von Aralkylhydroperoxiden wie z. B. Cumolhydroperoxid entstehen bereitzustellen, das die Herstellung eines salzarmen bzw. -freien und damit leichter aufarbeitbaren Spaltprodukts ermöglicht.

Es wurde nun überraschenderweise gefunden, daß die Entfernung von organischen und/oder anorganischen Säuren aus organischen Phasen durch Extraktion und eine anschließende Behandlung der organischen Phase mit einem Ionenaustauscher wesentlich vereinfacht und verbessert wird.

Gegenstand der vorliegenden Erfindung ist daher ein Verfahren zur Entfernung von organischen und/oder anorganischen Säuren aus organischen Phasen, das gekennzeichnet ist durch das Entfernen von organischen und/oder anorganischen Säuren mittels Extraktion und anschließender Behandlung der organischen Phase mit einem Ionenaustauscher.

Ebenfalls Gegenstand der vorliegenden Erfindung ist ein Verfahren zur Entfernung von organischen und/oder anorganischen Säuren aus Phasen, die bei der Spaltung von Aralkylhydroperoxiden entstehen, das gekennzeichnet ist durch das Entfernen von organischen und/oder anorganischen Säuren mittels Extraktion und anschließender Behandlung der Phasen mit einem Ionenaustauscher.

Mit Hilfe des erfindungsgemäßen Verfahrens können aus organischen und/oder anorganischen Säuren und anorganische Bestandteile enthaltenden organischen Phasen überraschend gut anorganische Bestandteile und Säuren entfernt werden. Ein Vorteil gegenüber den bekannten Verfahren besteht darin, daß im Extraktionsschritt nur etwa ein Viertel der Menge an Extraktionsmittel benötigt wird als bei den herkömmlichen Verfahren, da eine vollständige Entfernung der Säuren, wie dies bei den herkömmlichen Verfahren notwendig ist, nicht nötig ist. Wird im Extraktionsschritt Wasser als Extraktionsmittel eingesetzt, so erhält man im Vergleich zu den herkömmlichen Verfahren eine weniger verdünnte Schwefelsäure-Lösung, die aufgrund der Konzentration im Prozeß verwendet werden kann. Zur Extraktion wird vorzugsweise soviel Wasser eingesetzt, daß das zu extrahierende Gemisch mit Wasser gesättigt ist und zusätzlich bis zu 7 Gew.-%, vorzugsweise von 2 bis 7 Gew.-% und ganz besonders bevorzugt von 2 bis 4 Gew.-% Wasser, bezogen auf das Gemisch, zur Extraktion verwendet werden. Ein zusätzlicher wichtiger Effekt dieser Extraktion ist die Entfernung aller, aus den vorhergehenden Prozeßschritten eingetragener Salze. Der in der organischen Phase verbleibende Rest an Säure wird mit einem handelsüblichen Ionenaustauscher entfernt. Die Verwendung eines Ionenaustauschers hat den Vorteil, daß nicht nur der Anteil an anorganischen Säuren sondern auch der Anteil an organischen Säuren in der organischen Phase sehr effektiv verringert wird. In einer besonders bevorzugten Ausführungsart des Verfahrens im Zuge der Phenolherstellung aus Cumol kann zur Regeneration des eingesetzten Ionenaustauschers z.B. Phenolatlauge eingesetzt werden. Diese fällt im Gesamtprozeß der Phenolherstellung nach dem Cumolverfahren beim Auswaschen von Phenol aus dem Kopfprodukt der Cumol-Kolonne, welches hauptsächlich Cumol und $\alpha$-Methylstyrol enthält, mit Alkalilauge an. Durch Einsatz der Phenolatlauge zur Regeneration des Anionentauschers kann der Verbrauch von reinem Alkalihydroxid, welches üblicherweise zur Regeneration eines Anionenaustauschers eingesetzt wird, vermieden werden und somit ein erheblicher Kostenvorteil erzielt werden. Zusätzlich wird die Menge des Phenolats in der Phenolatlauge, die zur Rückgewinnung des Phenols angesäuert werden muß, geringer, da im Ionentauscher die Säure gegen das Phenol getauscht wird und somit schon ein Teil des Phenols zurückgewonnen wird. Dadurch, daß der organischen Phase vor der Behandlung im Ionenaustauscher ein großer Teil der Säuren durch Extraktion entzogen wird, wird der Ionenaustauscher, welcher nur zur Nachreinigung der organischen Phase eingesetzt wird, geringer belastet und hat damit eine längere Lebensdauer, welche sich positiv auf die Betriebskosten auswirkt.

Das erfindungsgemäße Verfahren wird im folgenden beispielhaft anhand der Spaltung von Aralkylhydroperoxiden, welche ein Zwischenprodukt der Hock'schen Synthese sind, insbesondere anhand der Spaltung von Cumolhydroperoxid, beschrieben, ohne darauf beschränkt zu sein.

Bei dieser in bekannter Weise durchgeführten Synthese wird aus Cumol und Sauerstoff Cumolhydroperoxid hergestellt. Dieses wird in einer Spaltreaktion säurekatalysiert in Phenol und Aceton aufgespalten. Als Katalysator wird Säure, zum Beispiel Schwefelsäure, verwendet. Die Spaltreaktion kann z.B. in homogener Phase durchgeführt werden. Die weitere beispielhafte Beschreibung des erfindungsgemäßen Verfahrens bezieht sich auf diese spezielle Durchführung der Spaltreaktion in homogener Phase mit einem Einsatz von 0,01 bis 2 Gew.-% Säure als Katalysator, ohne jedoch darauf beschränkt zu sein.

Die saure Spaltproduktphase, welche die als Katalysator verwendete Säure in einer Konzentration von z. B. 100 bis 1 000 ppm sowie organische Säuren, welche als Nebenprodukte bei der Oxidation von Cumol oder bei der säurekatalysierten Spaltung entstehen können, in einer Konzentration von ca. 300 ppm enthält, wird zum Entfernen der Säuren in einem ersten Schritt des erfindungsgemäßen Verfahrens z.B. unterhalb des Kopfes, vorzugsweise unmittelbar oberhalb des Sumpfes, in eine flüssig-flüssig Extraktionskolonne gefahren. Diese wird oberhalb des Sumpfes der Kolonne, vorzugsweise unmittelbar unterhalb des Kopfes der Kolonne mit einem Extraktionsmittel beschickt. Als Extraktionsmittel wird vorzugsweise Wasser eingesetzt. Die Einspeisung an Extraktionsmittel und zu extrahierender Phase in die Extraktionskolonne wird so gesteuert, daß bis zu 14 Gew.-% des Extraktionsmittels, vorzugsweise Wasser, bezogen auf das Spaltproduktgemisch in die Extraktionskolonne gefahren werden. Vorzugsweise wird soviel Wasser eingesetzt. daß das zu extrahierende Gemisch mit Wasser gesättigt ist und zusätzlich bis zu 7 Gew.-%, vorzugsweise von 2 bis 7 Gew.-% und ganz besonders bevorzugt von 2 bis 4 Gew.-% Wasser, bezogen auf das Gemisch, zur Extraktion verwendet werden. Das Extraktionsmittel und die zu extrahierende Phase durchströmen die Extraktionskolonne im Gleich- oder Gegenstrom, vorzugsweise im Gegenstrom.

Das mit Säure beladene Extraktionsmittel, bevorzugt Wasser, wird erfindungsgemäß vorzugsweise aus dem unteren Teil der Extraktionskolonne abgezogen. Im Fall von Schwefelsäure als Katalysator wird aus dem unteren Teil der Extraktionskolonne verdünnte Schwefelsäure abgezogen, die im Prozeß weiter verwendet werden kann.

Die Temperatur in der Extraktionsanlage liegt zwischen 0 und 90 °C, bevorzugt zwischen 20 und 50 °C und besonders bevorzugt zwischen 30 und 40 °C.

Der aus der Extraktionskolonne vorzugsweise aus dem Kopf erhaltene an wasserlöslichen Ionen abgereicherte Teil der organischen Spaltproduktphase, welche im hier betrachteten Beispiel noch organische Säuren in einer Konzentration von z. B. 180 bis 250 ppm sowie eine geringe Menge anorganischer Säuren (z.B. in einer Größenordnung von ca. 20 ppm) enthält, wird erfindungsgemäß über zumindest einen handelsüblichen Anionenaustauscher geleitet. Die Geschwindigkeit der Beladung des Anionenaustauschers hängt von der Dimensionierung des Austauscherbettes ab, welche wiederum von den zu behandelnden Volumenströmen und deren Säurekonzentrationen abhängig ist, und kann mittels Ventilen oder ähnlichem eingestellt werden.

Nach dem Durchlaufen des Anionenaustauschers ist die organische Spaltproduktphase soweit von organischen und anorganischen Säuren befreit, daß die Gesamtsäurekonzentration im angegebenen Beispielprozeß kleiner 10 ppm ist und der destillativen Aufarbeitung oder anderen Aufarbeitungsschritten zugeführt werden kann. Der Salzgehalt der organischen Spaltproduktphase beträgt nach Durchlaufen des Anionenaustauschers ebenfalls weniger als 10 ppm, berechnet als Natriumsulfat. Neben Extraktionskolonnen kann grundsätzlich (unabhängig vom Beispiel) jede kontinuierlich oder diskontinuierlich arbeitende Extraktionsvorrichtung eingesetzt werden. Als Anionenaustauscher können z.B. handelsübliche Polyacrylamid- bzw. Styroldivinylbenzolharze, wie z.B. VPOC 1072 oder MP 62 WS der Firma Bayer oder Amberlyst A 21 der Firma Rohm und Haas, verwendet werden.

Die erfindungsgemäße Regeneration des Anionenaustauschers kann mit Lauge, z.B. mit Alkalihydroxid-Lauge, erfolgen. Zum Zwecke der Regeneration wird die Regenerationslösung in gleicher Richtung oder in entgegengesetzter Richtung zur Richtung, in welcher die zu behandelnde Lösung den Anionenaustauscher durchströmt, vorzugsweise in gleicher Richtung, über den Anionenaustauscher gefahren. In einer besonders bevorzugten Ausführung des erfindungsgemäßen Verfahrens wird zur Regeneration des Anionenaustauschers freies Phenol enthaltende Phenolatlaugelösung, welche bei der Aufarbeitung von Phenol enthaltenden Phasen anfällt, genutzt.

Je nachdem, ob das Anionenaustauscherharz mit Alkalihydroxidlauge, z.B. mit NaOH, oder mit Phenolatlauge regeneriert wurde, entstehen im Anionenaustauschprozeß aus den in der organischen Phase enthaltenen Säuren Wasser oder Phenol gemäß der Formeln:

| Ionenaustauschprozeß | $Harz\text{-}OH + HX \rightarrow Harz\text{-}X + H_2O$ |
| Regeneration | $Harz\text{-}X + NaOH \rightarrow Harz\text{-}OH + NaX$ |
| Ionenaustauschprozeß | $Harz\text{-}Phenolat + HX \rightarrow Harz\text{-}X + Phenol$ |
| Regeneration | $Harz\text{-}X + Na\text{-}Phenolat \rightarrow Harz\text{-}Phenolat + NaX$ |

(X kennzeichnet einen anorganischen oder organischen Säurerest wie z. B. $HSO_4^-$ oder $Acetat^-$)

Entsteht im Ionenaustauschprozeß Wasser, so kann dieses durch z. B. einen Phasenabscheider von der organischen Phase getrennt werden. Entsteht im Ionenaustauschprozeß des besonders bevorzugten Verfahrens Phenol, so kann dieses direkt mit der organischen Spaltproduktphase der Aufarbeitung zugeführt werden.

Um das erfindungsgemäße Verfahren kontinuierlich betreiben zu können, kann es vorteilhaft sein, zumindest zwei Anionenaustauscher einzusetzen. Diese werden vorzugsweise in der Weise angeordnet, daß während der Beladung des einen Anionenaustauschers der andere in der oben beschriebenen Weise regeneriert werden kann und umgekehrt. Das Umstellen der Anionenaustauscher von Regeneration auf Ionenaustausch und umgekehrt, kann mit Hilfe von Ventilen, Absperrhähnen oder ähnlichem auf eine dem Fachmann bekannten Weise erfolgen.

Erfindungsgemäß kann die Verwendung mehrerer Extraktionskolonnen oder mehrerer Ionenaustauscher oder die Verwendung mehrerer Kombinationen von Extraktionskolonnen mit nachgeschalteten Ionenaustauschern vorgesehen werden.

Das erfindungsgemäße Verfahren wird vorzugsweise bei Umgebungsdruck und bei Temperaturen zwischen 0 und 90 °C, bevorzugt bei Temperaturen zwischen 20 und 50 °C und besonders bevorzugt bei Temperaturen zwischen 30 und 40 °C ausgeführt. Erfindungsgemäß kann als Extraktionsmittel saures oder neutrales Wasser, vorzugsweise neutrales Wasser verwendet werden.

Das erfindungsgemäße Verfahren kann kontinuierlich oder diskontinuierlich ausgeführt werden.

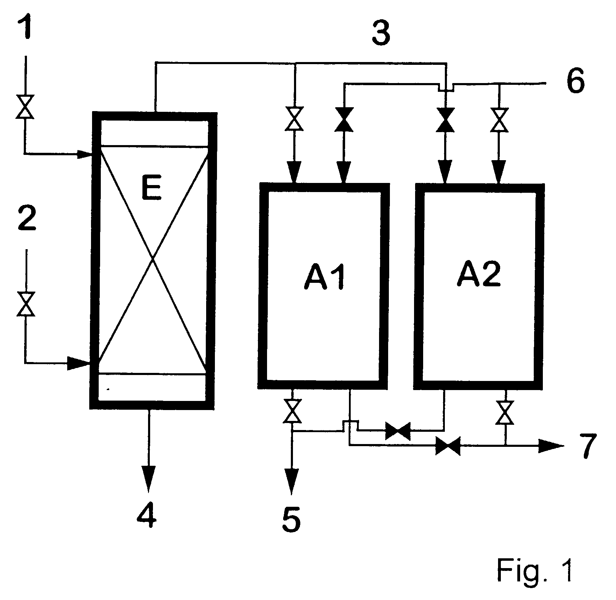

In Figur 1 ist beispielhaft eine Ausführungsart des erfindungsgemäßen Verfahrens dargestellt, ohne daß das Verfahren auf diese beschränkt ist.

Das zu behandelnde Spaltgemisch **2** wird von unten in den Extraktor **E** gefahren. Von oben wird in den Extraktor **E** Wasser **1** gefahren. Die ausgewaschene Säure **4** verläßt den Extraktor **E** am Fuß der Extraktionsanlage. Die von einem großen Teil der Säure befreite organische Phase **3** wird im oberen Teil der Extraktionsanlage **E** abgenommen und wird entweder über den Anionenaustauscher **A1** oder über den Anionenaustauscher **A2** geleitet. Nach dem Durchlaufen des Anionentauschers steht die zumindest nahezu säurefreie organische Phase der weiteren Verwendung zu Verfügung.

Während die organische Phase den Anionenaustauscher **A1** durchläuft und die säurefreie organische Phase **5** nach Verlassen des Anionenaustauschers der Weiterverarbeitung zugeführt wird, kann der andere Anionenaustauscher **A2** mit z.B. einer Natriumphenolatlösung **6** regeneriert werden. Die dann bei der Regeneration des Anionentauschers entstehende salzhaltige, wäßrige Phase **7** wird einer Aufarbeitung oder einer Entsorgung zugeführt.

Das erfindungsgemäße Verfahren wird durch das folgende Beispiel näher erläutert, ohne darauf beschränkt zu sein.

Beispiel 1:

Entfernung von Schwefelsäure und organischen Säuren aus Gemischen, die bei der einphasigen Spaltung von Cumolhydroperoxid entstehen

Bei der Gewinnung von Phenol und Aceton ausgehend von Cumol nach dem klassischen Hock-Verfahren fällt bei der durch Schwefelsäure katalysierten Spaltreaktion von Cumolhydroperoxid zu Phenol und Aceton ein saures Spaltgemisch an, welches in diesem Beispiel 740 ppm Schwefelsäure enthält. Hinzu kommen 207 ppm organische Säuren, so daß der Gesamtsäuregehalt (berechnet als Schwefelsäure) 961 ppm beträgt.

Ein Teil dieses Spaltgemisches wurde durch eine Extraktionskolonne gefahren. Zusätzlich wurden 7 Gew.-% Wasser in die Extraktionsanlage gefahren. Nach erfolgter Extraktion bei einer Temperatur von 25 °C betrug der Schwefelsäuregehalt in der organischen Phase < 10 ppm. Der Gesamtsäuregehalt in der organischen Phase betrug nach erfolgter Extraktion noch 201 ppm (berechnet als Schwefelsäure), da sich die organischen Säuren kaum extrahieren ließen.

Diese organische Phase wurde über einen Anionenaustauscher, der mit dem handelsüblichen Anionenaustauscherharz VPOC 1072 der Firma Bayer AG gefüllt war, gefahren: Die Temperatur der organischen Phase betrug 25 °C. Nach dem Durchlaufen des Anionentauschers wurde die behandelte organische Phase analysiert und es ließ sich Säure nur noch in einer Konzentration von < 5 ppm, berechnet als $H_2SO_4$, in der organischen Phase feststellen.

**Patentansprüche**

1. Verfahren zur Entfernung von organischen und/oder anorganischen Säuren aus einer organischen Phase, gekennzeichnet durch Entfernen der organischen und/oder anorganischen Säuren mittels Extraktion und anschließender Behandlung der organischen Phase mit einem Ionenaustauscher.

2. Verfahren zur Entfernung von organischen und/oder anorganischen Säuren aus Phasen, die bei der einphasigen Spaltung von Aralkylhydroperoxiden entstehen, gekennzeichnet durch Entfernen der organischen und/oder anorganischen Säuren mittels Extraktion und anschließender Behandlung der organischen Phase mit einem Ionenaustauscher.

3. Verfahren nach Anspruch 2,
   dadurch gekennzeichnet,
   daß zur Extraktion bis zu 14 Gew.-% Wasser, bezogen auf das Gemisch, verwendet werden.

4. Verfahren nach zumindest einem der Ansprüche 1 oder 2,
   dadurch gekennzeichnet,
   daß zur Extraktion eines mit Wasser gesättigten Gemisches zusätzlich bis zu 7 Gew.-% Wasser, bezogen auf das Gemisch, verwendet werden.

5. Verfahren nach Anspruch 4,
   dadurch gekennzeichnet,

daß zur Extraktion eines mit Wasser gesättigten Gemisches zusätzlich von 2 bis 7 Gew.-% Wasser, bezogen auf das Gemisch, verwendet werden.

6. Verfahren nach Anspruch 5,
   dadurch gekennzeichnet,
   daß zur Extraktion eines mit Wasser gesättigten Gemisches zusätzlich von 2 bis 4 Gew.-% Wasser, bezogen auf das Gemisch, verwendet werden.

7. Verfahren nach zumindest einem der Ansprüche 1 bis 6,
   dadurch gekennzeichnet,
   daß die Temperatur zwischen 10 und 90 °C liegt.

8. Verfahren nach Anspruch 7,
   dadurch gekennzeichnet,
   daß die Temperatur zwischen 20 und 50 °C liegt.

9. Verfahren nach Anspruch 8,
   dadurch gekennzeichnet,
   daß die Temperatur zwischen 30 und 40 °C liegt.

10. Verfahren nach zumindest einem der Ansprüche 1 bis 9,
    dadurch gekennzeichnet,
    daß zur Regeneration des Anionenaustauschers Alkalihydroxid verwendet wird.

11. Verfahren nach zumindest einem der Ansprüche 1 bis 10,
    dadurch gekennzeichnet,
    daß zur Regeneration des Anionenaustauschers Phenolatlauge verwendet wird.

Fig. 1

**Europäisches Patentamt**

## EUROPÄISCHER RECHERCHENBERICHT

Nummer der Anmeldung

EP 99 11 4766

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | DATABASE WPI Section Ch, Derwent Publications Ltd., London, GB; Class E14, AN 72-67993T XP002116040 & DD 91 643 A (BOHME G KIESSLING W MOLL) * Zusammenfassung * | 1-11 | C07C45/53 C07C45/80 C07C45/79 C07C37/08 C07C37/72 C07C37/82 |
| Y | CHEMICAL ABSTRACTS, vol. 113, no. 1, 2. Juli 1990 (1990-07-02) Columbus, Ohio, US; abstract no. 005932, GUMEROVA N M ET AL: "Method of extracting sulfuric acid catalyst from isopropylbenzene hydroperoxide decomposition residue" XP000063523 * Zusammenfassung * & SU 1 544 761 A (SCIENTIFIC-RESEARCH INSTITUTE OF PETROCHEMICAL PRODUCTION;USSR) | 1-11 | |
| Y | GB 649 286 A (THE DISTILLERS COMPANY LIMITED) 24. Januar 1951 (1951-01-24) * Seite 2, Spalte 2, Zeile 68 - Zeile 74; Anspruch 1 * | 1-11 | RECHERCHIERTE SACHGEBIETE (Int.Cl.7) C07C |
| D,Y | FR 1 302 848 A (SOCIETA' ITALIANA RESINE) 2. Januar 1963 (1963-01-02) * Seite 1, Spalte 2 - Seite 2, Spalte 1; Ansprüche * | 1-11 | |

-/--

Der vorliegende Recherchenbericht wurde für alle Patentansprüche erstellt

| Recherchenort | Abschlußdatum der Recherche | Prüfer |
|---|---|---|
| DEN HAAG | 21. September 1999 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument. das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie.übereinstimmendes Dokument

EPO FORM 1503 03.82 (P04C03)

Europäisches
Patentamt

**EUROPÄISCHER RECHERCHENBERICHT**

Nummer der Anmeldung

EP 99 11 4766

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | CHEMICAL ABSTRACTS, vol. 091, no. 15, 8. Oktober 1979 (1979-10-08) Columbus, Ohio, US; abstract no. 123553, BOGDANIAK-SULINSKA W ET AL: "Regeneration of anion exchangers in the neutralization of an acid mixture of phenol and acetone" XP002116039 * Zusammenfassung * & PL 99 853 - (INSTYTUT CHEMII PRZEMYSLOWEJ;POL.) ----- | 1-11 | |
| | | | **RECHERCHIERTE SACHGEBIETE (Int.Cl.7)** |

Der vorliegende Recherchenbericht wurde für alle Patentansprüche erstellt

| Recherchenort | Abschlußdatum der Recherche | Prüfer |
|---|---|---|
| DEN HAAG | 21. September 1999 | Bonnevalle, E |

## ANHANG ZUM EUROPÄISCHEN RECHERCHENBERICHT
## ÜBER DIE EUROPÄISCHE PATENTANMELDUNG NR.

EP 99 11 4766

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-09-1999

| Im Recherchenbericht angeführtes Patentdokument | Datum der Veröffentlichung | Mitglied(er) der Patentfamilie | Datum der Veröffentlichung |
|---|---|---|---|
| DD 91643 A | | KEINE | |
| SU 1544761 A | 23-02-1990 | KEINE | |
| GB 649286 A | | KEINE | |
| FR 1302848 A | 02-01-1963 | KEINE | |
| PL 99853 - | | KEINE | |

EPO FORM P0461

Für nähere Einzelheiten zu diesem Anhang : siehe Amtsblatt des Europäischen Patentamts. Nr. 12/82